# EUROPEAN PATENT APPLICATION

(11) **EP 1 371 652 A1**
(43) Date of publication of application: **17.12.2003**
(21) Application number: 02705393.3
(22) Date of filing: 20.03.2002
(51) Int. Cl.: C07D 471/04, C09D 11/00, B41J 2/01, C07F 5/00

(54) **EUROPIUM COMPOUNDS AND INK COMPOSITIONS CONTAINING THE SAME**

(30) Priority: 22.03.2001 JP 2001082060
(71) Applicant: Nippon Kayaku Kabushiki Kaisha, Tokyo 102-8172 (JP)
(72) Inventor: IKEDA, Masaaki, Nippon Kayaku Kabushiki Kaisha, Tokyo 115-0042 (JP); YOSHIOKA, Junko, Nippon Kayaku Kabushiki Kaisha, Tokyo 115-0042 (JP); SHIRASAKI, Yasuo, Nippon Kayaku Kabushiki Kaisha, Tokyo 115-0042 (JP); KIYOYANAGI, Noriko, Kita-ku, Tokyo 115-0052 (JP); KITAYAMA, Yasuyuki, Saitama-shi, Saitama 330-0835 (JP)
(74) Representative: Wablat, Wolfgang, Dr.Dr.
(86) International application number: JP0202678
(87) International publication number: WO02079201

(57) **Abstract**

Europium compounds represented by the general formula (1) and water-base ink compositions containing the same: 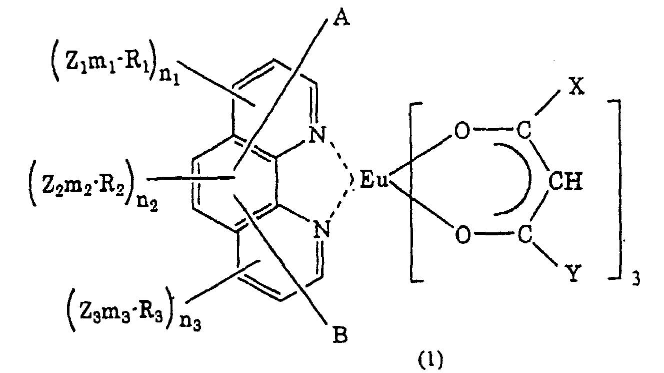 [wherein X is an optionally substituted aromatic or heterocyclic group: Y is a fluorohydrocarbon group having 1 to 10 carbon atoms: Z₁ to Z₃ are each independently a hydrophilicity-imparting group: R₁ to R₃ are each a direct bond or a divalent hydrocarbon or heterocyclic group which may be substituted: n₁ to n₃ are each independently an integer of 0 to 2: and m₁ to m₃ are each independently an integer of 1 to 3, with the proviso that all of n₁ to n₃ must not be 0]. The compounds and the ink compositions are excellent in stability and safety, and emit red light by irradiation with ultraviolet light though they are substantially invisible in the visible light region. Thus, they are useful for prints which are to be read by a system having a sensor reading function with the need for hidden letters or security markings.

## Description

### Technical Field

The present invention relates to a novel europium compound and uses thereof. In particular, the present invention relates to a compound which is not recognizable with the naked eye in visible rays but is recognizable due to coloration upon irradiation with UV rays and to an ink composition containing the same.

### Background Art

There are known compounds such as a tris(thenoyltrifluoroacetonate)europium complex and a tris(benzoyltrifluoroacetonate)europium complex which are colorless and visually unrecognizable in visible rays but are recognizable or readable by emitting red light upon irradiation with UV rays, and application of these compounds to various inks etc. is attempted. However, coloring matters conventionally used for this object are poor in light resistance and inferior in solubility in water, and solutions thereof are poor in stability with time. Accordingly, there is a limit to application thereof. For example, Japanese Patent Application Laid-Open No. 8-253715 and Japanese Patent Application Laid-Open No. 8-239609 describe application of a europium complex to a water-containing solvent, but at present, the sufficient solubility thereof in a solvent, particularly in water, cannot be achieved.

### Disclosure of Invention

The object of the present invention is to provide a novel compound which is colorless and not recognizable with the naked eye in visible rays, but is colored upon irradiation with UV rays, is highly soluble in a solvent particularly in a water-containing solvent, and is excellent in the stability of the solutions.

The present inventors made extensive study for solving the above-described problem, and as a result, they found that a europium compound (complex) having a specific structure having a phenanthroline nucleus is excellent in solubility in an aqueous solvent, and the present invention was thereby arrived at.

That is, the present invention relates to:
(1) a europium compound represented by formula (1): 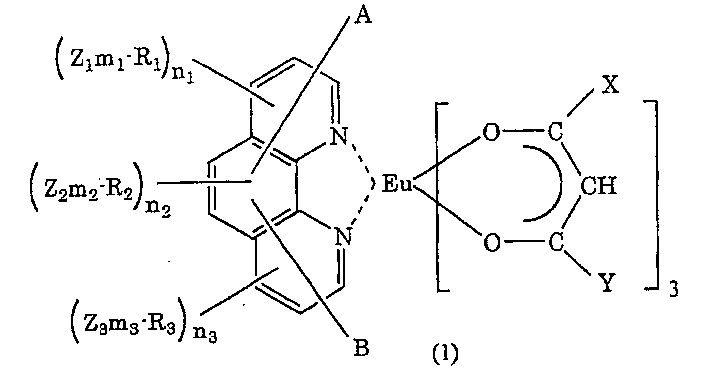 wherein X represents an aromatic or heterocyclic group which may have a substituent group, Y represents a fluorohydrocarbon group having 1 to 10 carbon atoms, A and B independently represent a substituent group, Z₁ to Z₃ independently represent a hydrophilicity-imparting group, R₁ to R₃ are each independently a direct bond or an optionally substituted divalent hydrocarbon group or heterocyclic group, n₁ to n₃ are each independently an integer of 0 to 2, and m₁ to m₃ are each independently an integer of 1 to 3, with the proviso that all of n₁ to n₃ are not simultaneously 0,
(2) the europium compound according to the above-mentioned (1) , wherein each of Z₁ to Z₃ is a group selected from the group consisting of a sulfonic acid group, a carboxyl group, a hydroxyl group and a phosphonic acid group,
(3) the europium compound according to the above-mentioned (1) or (2), wherein each of R₁ to R₃ is a phenyl group,
(4) the europium compound according to any of the above-mentioned (1) to (3), wherein each of Z₁ to Z₃ is a sulfonic acid group, and each of R₁ to R₃ is a phenyl group,
(5) a europium compound represented by formula (2): 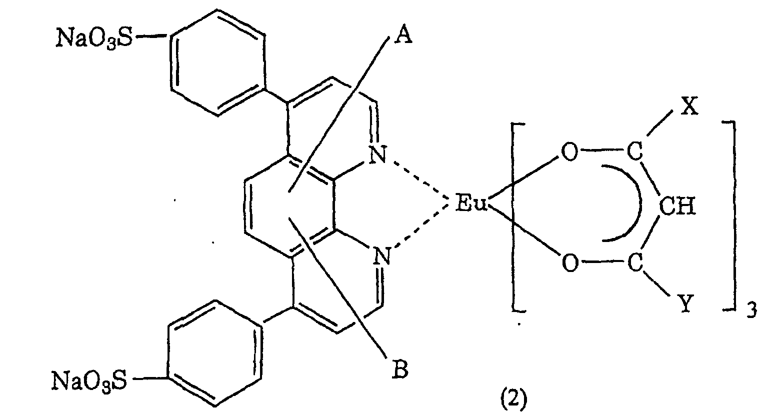 wherein X represents an aromatic or heterocyclic group which may have a substituent group, Y represents a fluorohydrocarbon group having 1 to 10 carbon atoms, and A and B independently represent a substituent group,
(6) the europium compound according to the above-mentioned (5) , wherein in the formula (2), X is a group selected from the group consisting of a benzene ring group, a naphthalene ring group, a pyridine ring group, a thiophene ring group and a furan ring group, each of which may have a substituent group,
(7) the europium compound according to the above-mentioned (5) or (6), wherein in the formula (2), Y is a trifluoromethyl group,
(8) the europium compound according to the above-mentioned (5), wherein in the formula (2), the substituent groups A and B are each independently a hydrogen atom or a hydrocarbon group having 1 to 8 carbon atoms which may have a substituent group,
(9) a europium compound represented by formula (3) : 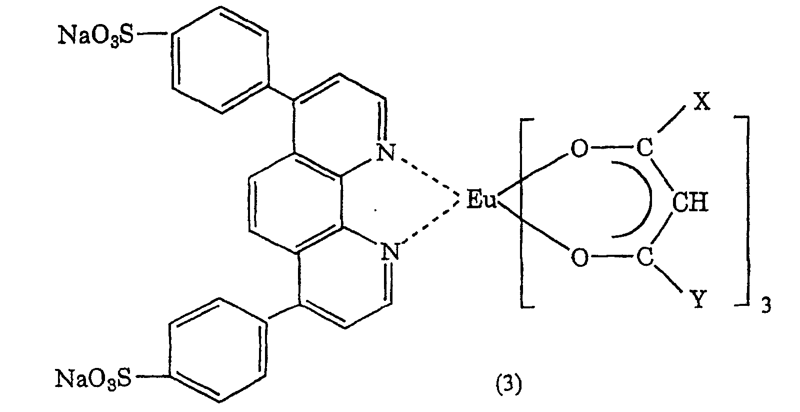 wherein X represents an aromatic or heterocyclic group which may have a substituent group, and Y represents a fluorohydrocarbon group having 1 to 10 carbon atoms,
(10) the europium compound according to the above-mentioned (9), wherein in the formula (3), X is a group selected from the group consisting of a benzene ring group, a naphthalene ring group, a pyridine ring group, a thiophene ring group and a furan ring group, each of which may have a substituent group,
(11) an aqueous ink composition comprising a water-soluble europium compound (complex) obtained from a water-soluble phenanthroline derivative, a propanedione derivative and a europium compound,
(12) an aqueous ink composition, which comprises the europium compound described in any of the above-mentioned (1) to (10),
(13) the aqueous ink composition according to the above-mentioned (11) or (12), which comprises an organic solvent,
(14) the aqueous ink composition according to any of the above-mentioned (11) to (13), which comprises a polymer having a cyclic amido group,
(15) the aqueous ink composition according to the above-mentioned (14), wherein the polymer having a cyclic amido group is at least one member selected from the group consisting of polyvinyl caprolactam, a vinyl pyrrolidone/vinyl caprolactam copolymer, a vinyl pyrrolidone/vinyl imidazole copolymer, polyvinyl pyrrolidone, and a vinyl pyrrolidone/vinyl acetate copolymer,
(16) the aqueous ink composition according to the above-mentioned (11) or (13), which comprises a carboxyl group- or sulfonic acid group-modified polyvinyl alcohol,
(17) the aqueous ink composition according to any of the above-mentioned (11) to (16), which is used in ink-jet recording,
(18) an ink-jet recording method for recording a recording material by discharging ink droplets depending on record signal, characterized by using the aqueous ink composition described in any of the above-mentioned (11) to (17),
(19) the ink-jet recording method according to the above-mentioned (18), wherein the recording material is an information transmission sheet, and
(20) the ink-jet recording method according to the above-mentioned (19), wherein the information transmission sheet is a surface-treated sheet.

### Brief Description of Drawings

Fig. 1 is a fluorescence spectrum of a mark recorded by ink jetting on an ordinary paper in Example 3 and taken with a fluorospectrophotometer FP-6600 manufactured by JASCO Corporation.
Fig. 2 is a fluorescence spectrum of a mark recorded by ink jetting on special paper A in Example 3 and taken with a fluorospectrophotometer FP-6600 manufactured by JASCO Corporation.

### Best Mode for Carrying Out the Invention

The present invention is described in detail.

The europium compound (complex) of the present invention is represented by the formula (1) above, and X in the formula (1) includes an aromatic ring or heterocyclic ring which may have a substituent group, and the aromatic group includes, for example, a benzene ring, a naphthalene ring, an anthracene ring, an azulene ring, a phenanthrene ring etc., and the heterocyclic ring includes, for example, a pyridine ring, a thiophene ring, a furan ring, a pyrazine ring, a thiazole ring, an oxazole ring, a quinoline ring, an indole ring etc. Preferable among those are a benzene ring, a naphthalene ring, a pyridine ring, a thiophene ring, a furan ring etc. Particularly, the compound having a naphthalene ring is preferable because of high emission intensity. The substituent group which may be possessed by X includes a hydrogen atom, an alkyl group, an alkoxyl group, an aromatic group, a heterocyclic group, an amino group, an alkylamino group, a dialkylamino group, a hydroxyl group, an aralkyl group, a halogen atom etc. Preferable among those is a hydrogen atom, a halogen atom or an alkyl group.

Y represents a fluorohydrocarbon group having 1 to 10 carbon atoms, and examples thereof include perfluoroalkyl groups such as trifluoromethyl group, pentafluoroethyl group, heptafluoropropyl group, heptadecafluorooctyl group etc. and monofluoromethyl group, difluoromethyl group, trifluoroethyl group, a tetrafluoropropyl group, octafluoropentyl group etc. Preferable among those are perfluoroalkyl groups such as trifluoromethyl group, pentafluoroethyl group, heptadecafluorooctyl group etc., more preferably trifluoromethyl group.

Z₁ to Z₃ in the formula (1) independently represent a hydrophilicity-imparting group. Examples of the hydrophilicity-imparting group include hydroxyl group, carboxyl group, sulfonic acid group and phosphonic acid group, preferably hydroxyl group, carboxylic acid group or sulfonic acid group, more preferably sulfonic acid group. Each of the carboxyl group, sulfonic acid group and phosphonic acid group may be a free acid, or may have formed a salt with a metal ion such as lithium, sodium or potassium or with a cation such as a quaternary ammonium salt as counterions. Further, when each of R₁ to R₃ has two or more substitutable sites, each of Z₁ to Z₃ has a plurality of hydrophilicity-imparting groups which may be the same or different.

In the formula (1), R₁ to R₃ each independently represent a direct bond or an optionally substituted divalent hydrocarbon group or heterocyclic group. The optionally substituted divalent hydrocarbon group or heterocyclic group include a saturated or unsaturated alkyl group such as -CH₂-, -C₂H₄-, -C₅H₁₁-, -C₈H₁₆-, -CH=CH-, -CH=CH-CH₂- or -C≡C-CH₂-, a saturated or unsaturated cyclic alkyl group such as cyclohexylene or cyclohexenylene, an aromatic hydrocarbon group such as a phenylene group or naphthylene group, and pyridinediyl, quinolinediyl etc. Preferable examples include a saturated or unsaturated alkyl group such as -CH₂-, -C₂H₄-, -C₅H₁₁-, -C₈H₁₆-, -CH=CH-, -CH=CH-CH₂- or -C≡C-CH₂-, and an aromatic hydrocarbon group such as a phenylene group or naphthylene group, and more preferable examples include an aromatic hydrocarbon group such as a phenylene group. The substituent group includes hydrogen atom, alkyl group, alkoxyl group, aromatic group, heterocyclic group, amino group, alkylamino group, dialkylamino group, hydroxyl group, aralkyl group, halogen atom etc. Preferable examples include hydrogen atom, halogen atom and alkyl group.

In the formula (1), A and B each independently represent a substituent group. Examples of the substituent group include a hydrogen atom, an optionally substituted hydrocarbon group having 1 to 8 carbon atoms, a nitro group, a cyano group, a halogen atom, hydroxyl group etc. Preferable examples include hydrogen atom, methyl group, ethyl group, propyl group, butyl group, phenyl group and tolyl group.

The europium compound of formula (1) in the present invention is produced for example by the following method.

As shown in the reaction scheme below, the europium compound can be easily synthesized by reacting, for example, a diketone derivative with a phenanthroline derivative having hydrophilicity-imparting groups, together with europium perchlorate or europium chloride, in the presence of sodium hydroxide in e.g. water or an alcohol or acetone solvent, preferably at 0°C to 80°C.

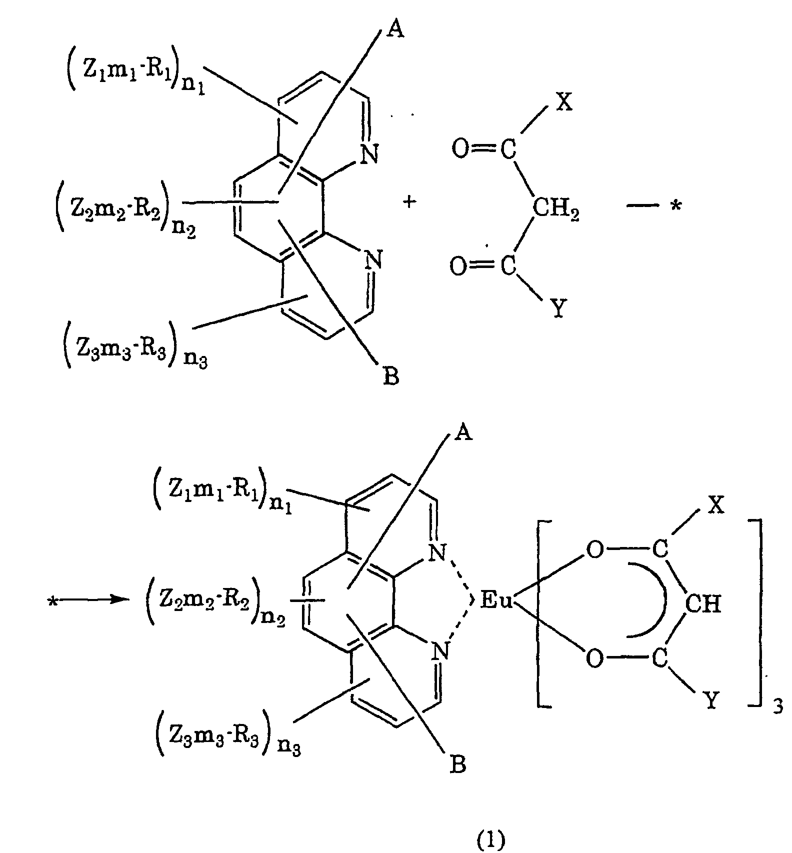

Examples of the europium compound of formula (1) in the present invention are shown below.

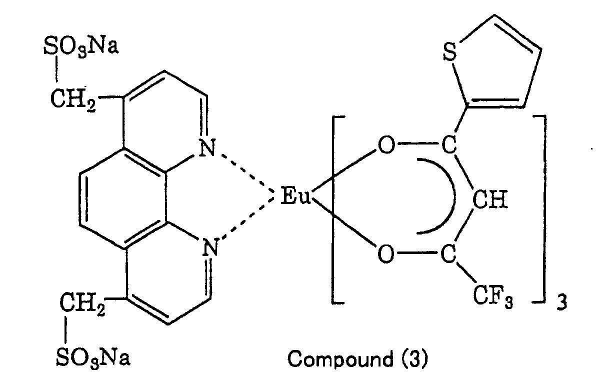

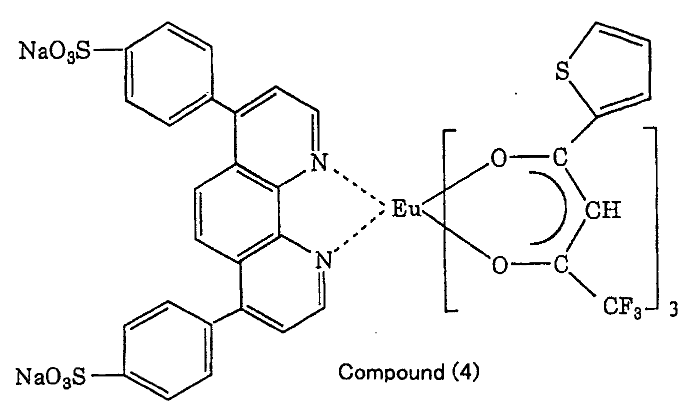

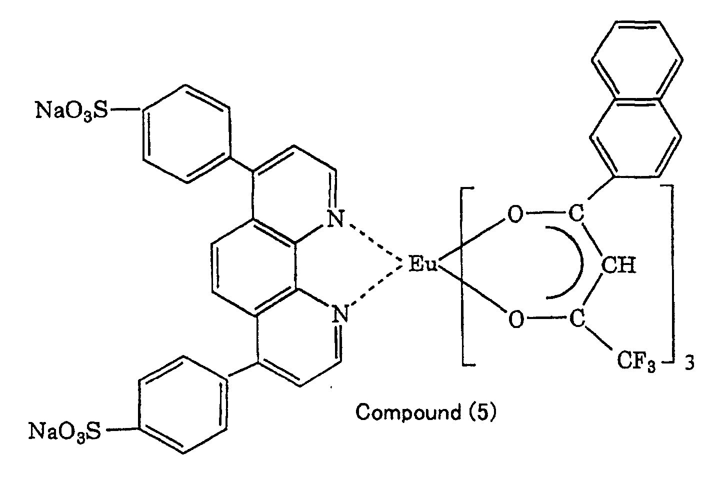

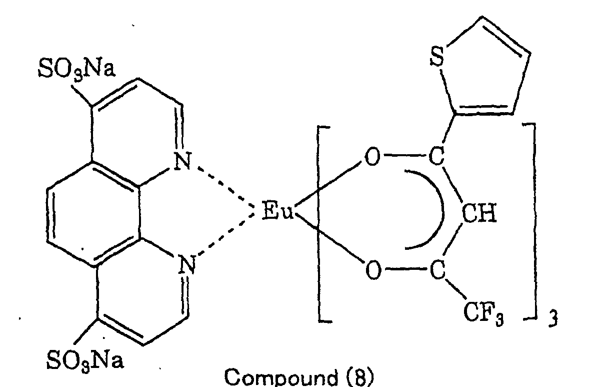

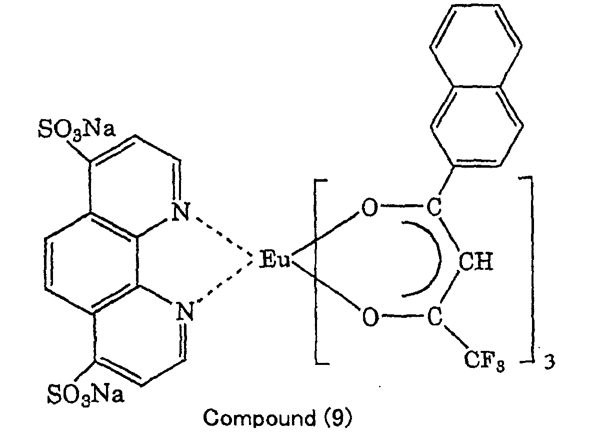

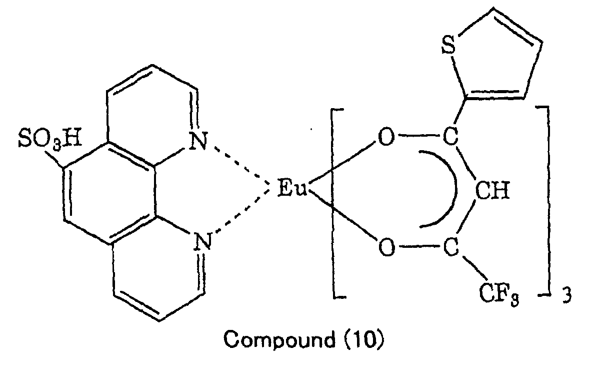

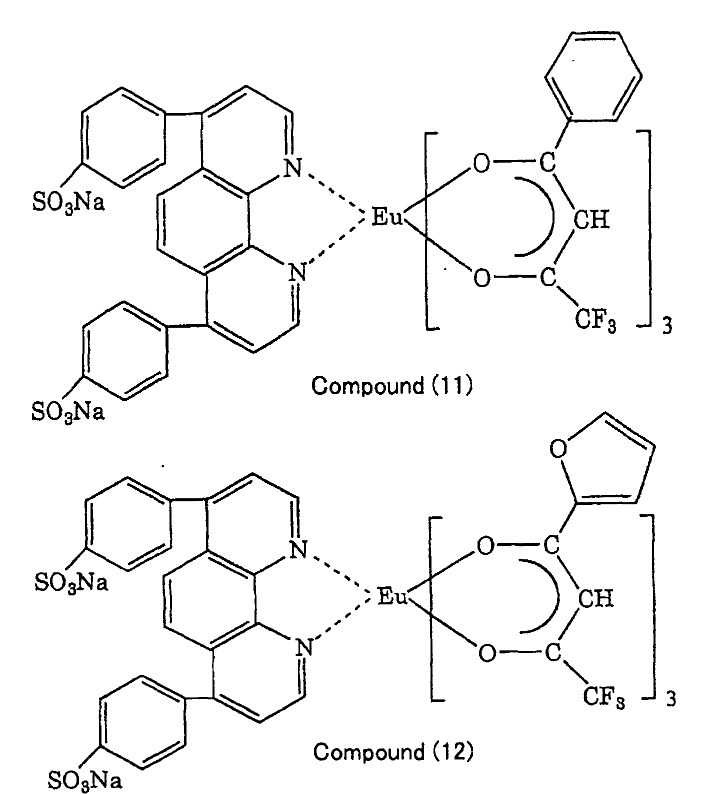

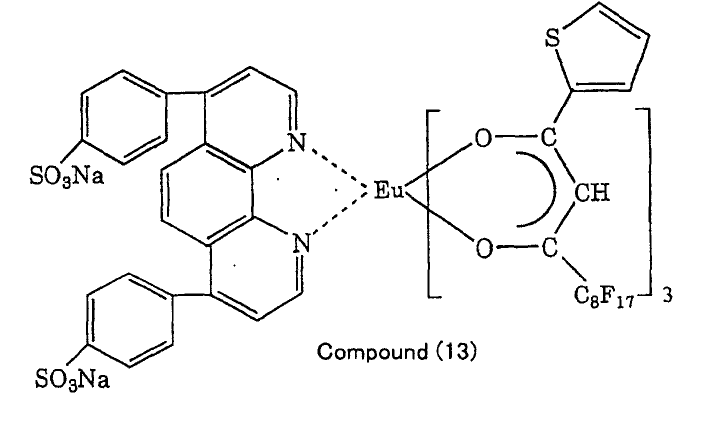

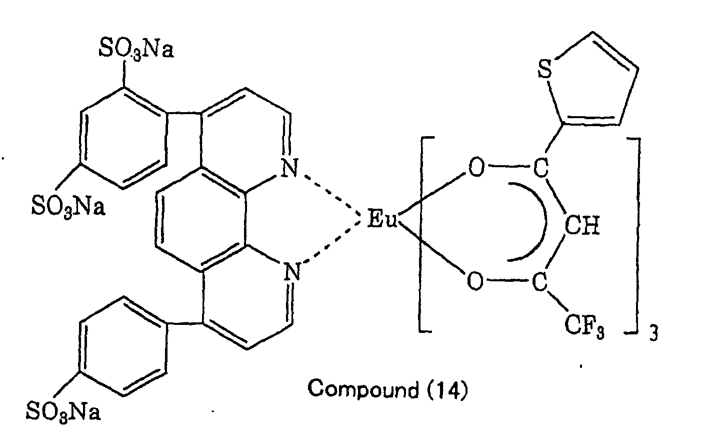

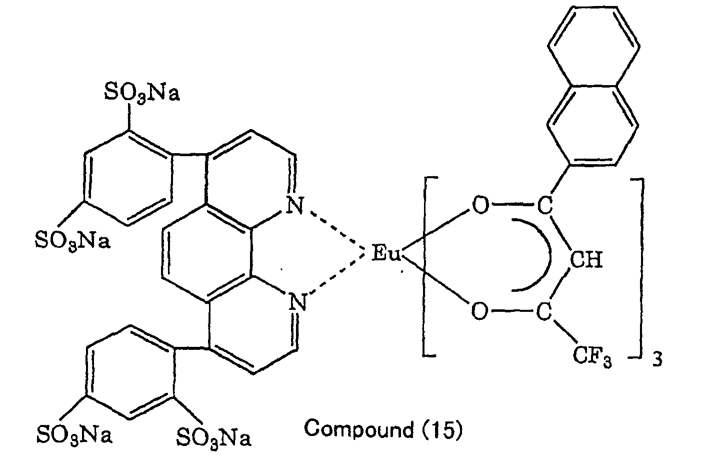

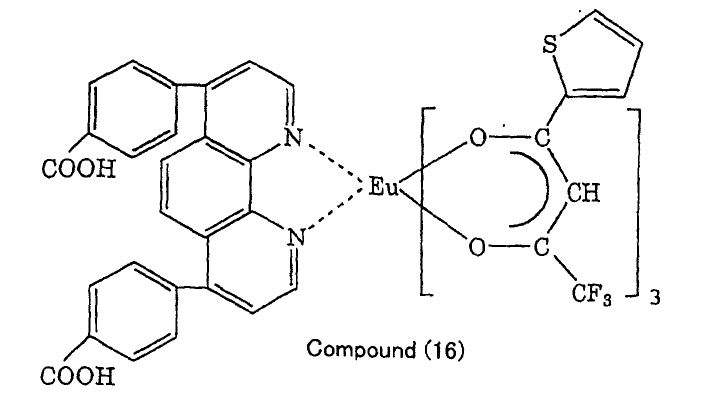

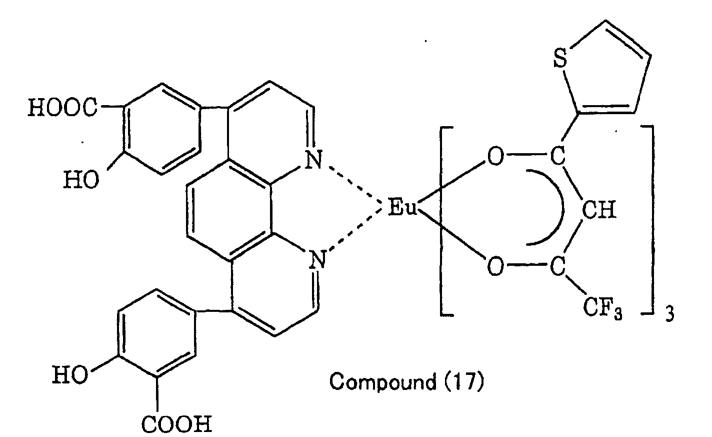

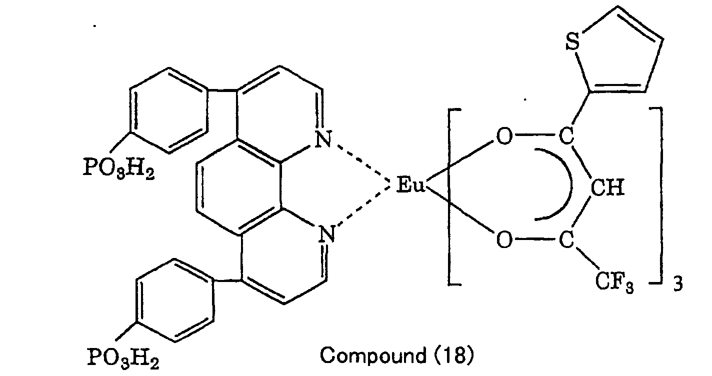

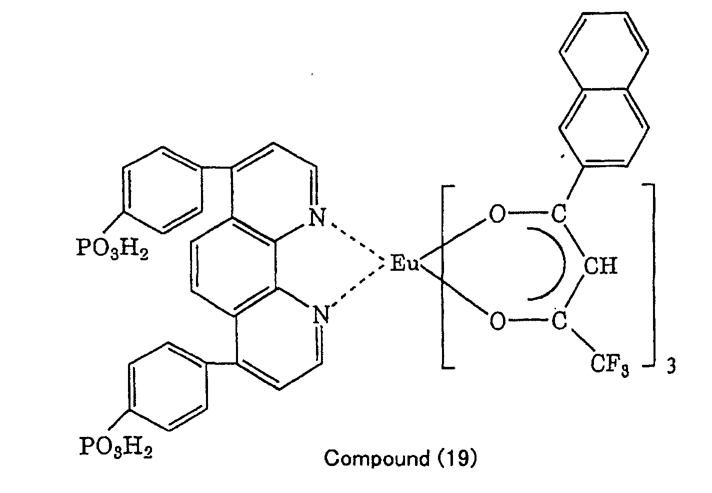

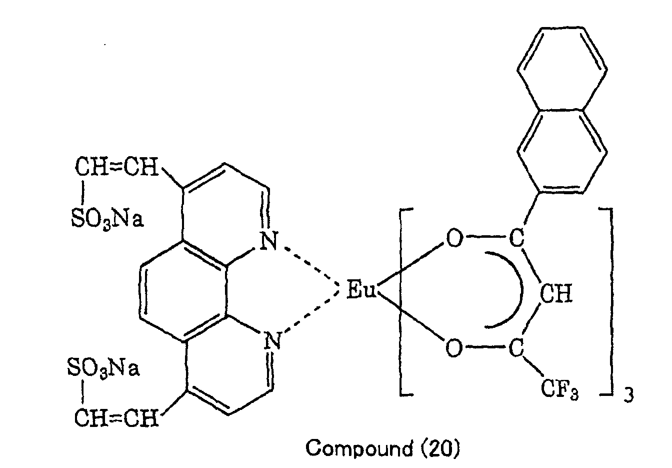

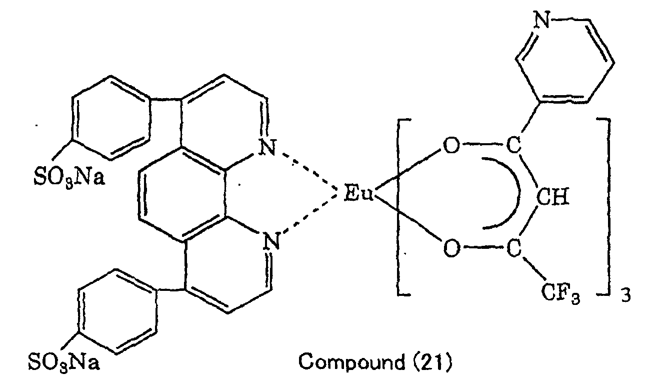

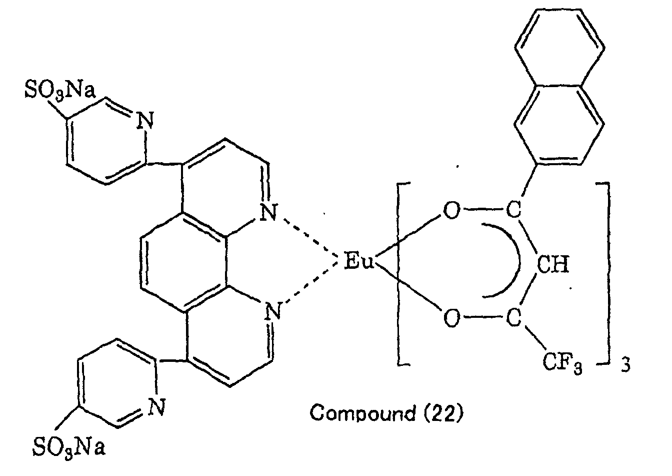

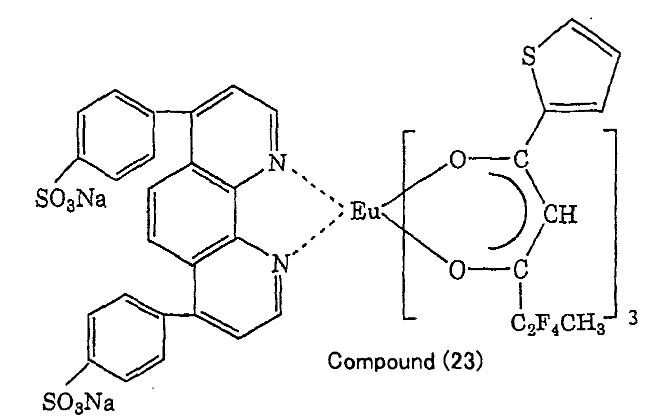

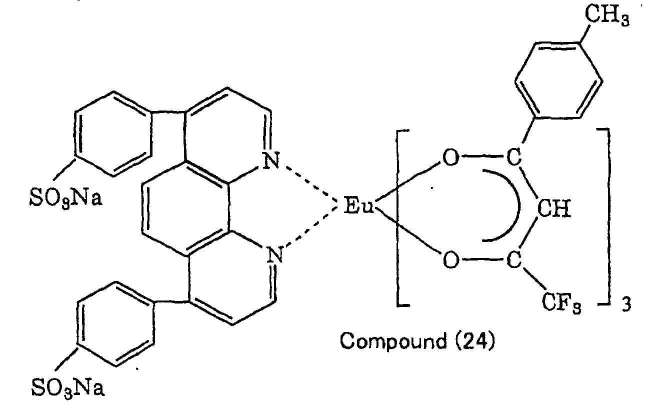

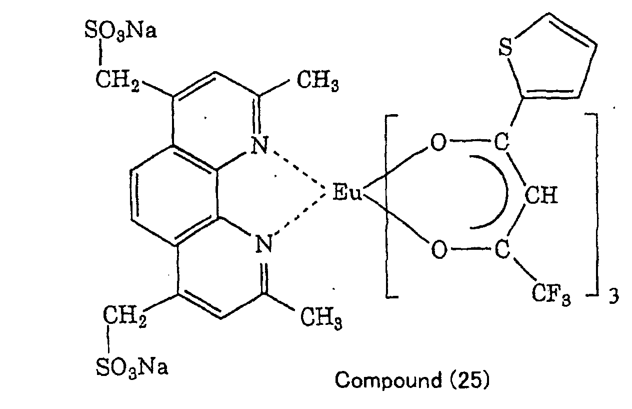

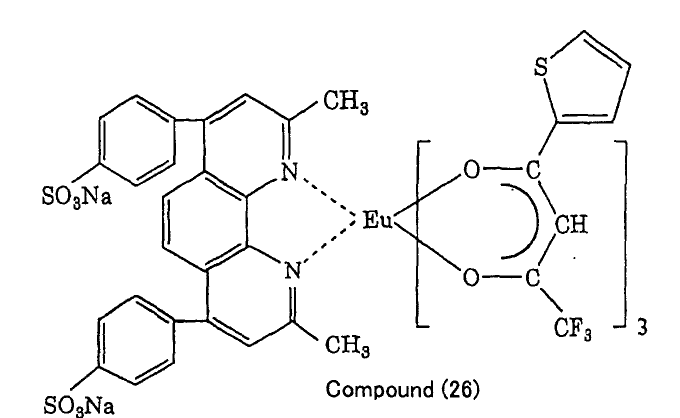

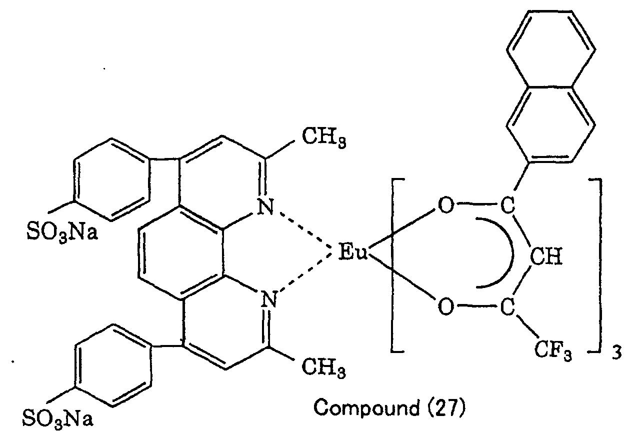

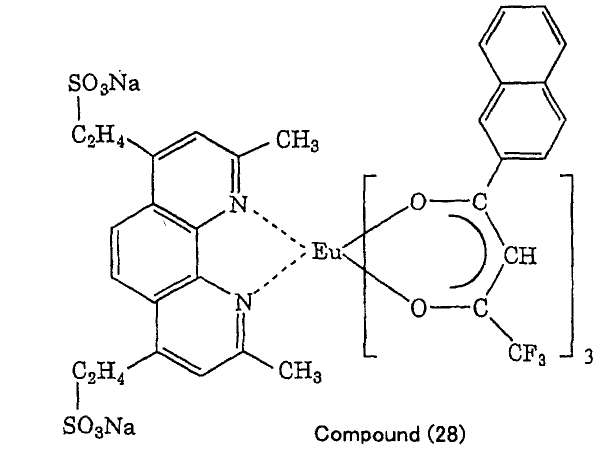

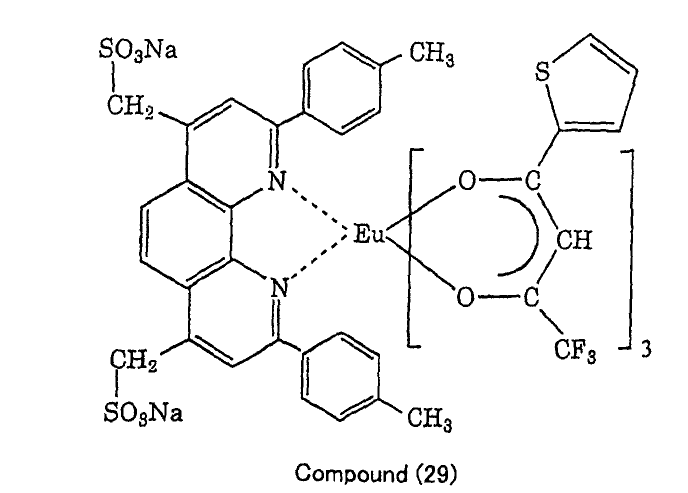

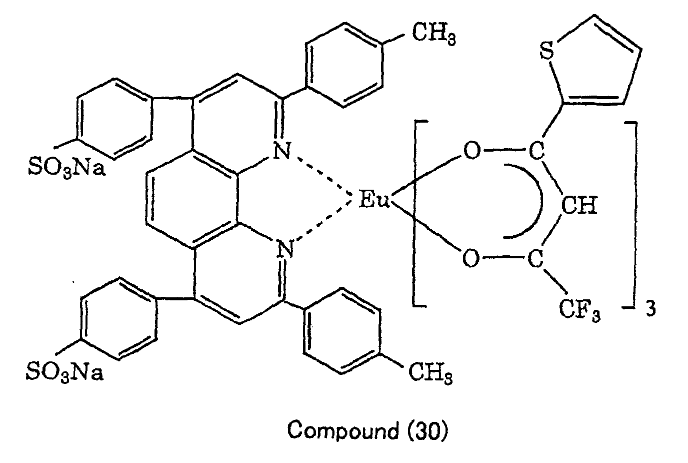

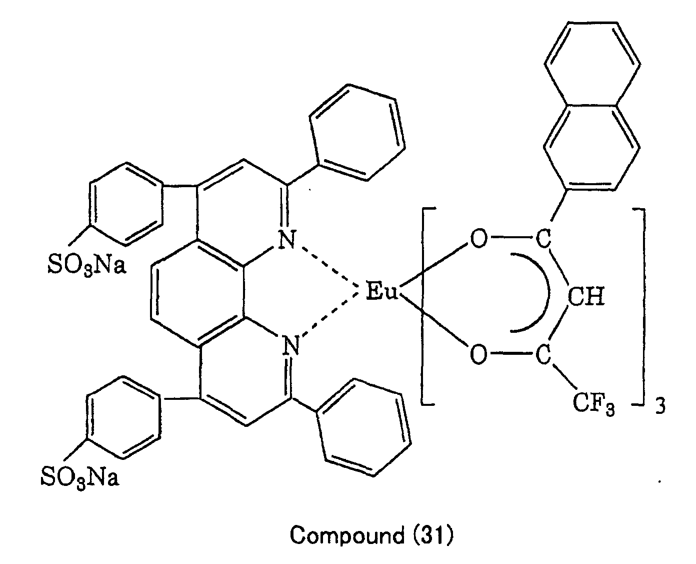

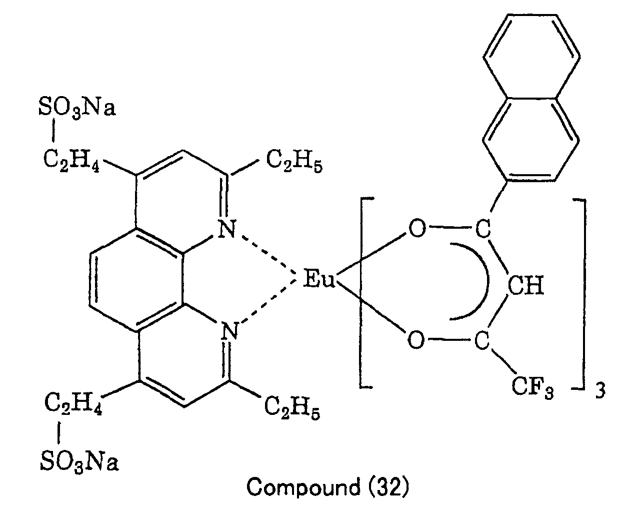

The europium compound represented by formula (1) in the present invention can be utilized as a molded product unrecognizable in visible rays but emitting red light upon irradiation with UV rays, which is produced by mixing the europium compound for example with a resin such as polystyrene or polyacrylate or with a monomer thereof, then polymerizing the mixture if necessary, and heating and melting it to form a resin plate or film, or by coating a base material with an ink composition containing the europium compound represented by formula (1) in the present invention, but preferable use of the europium compound represented by formula (1) in the present invention lies in utilization thereof as a recording material prepared as an ink composition containing the compound of the present invention. The ink composition of the present invention can be applied or printed on a paper, synthetic paper or film by a known method such as conventional bar-coater coating, air-knife coating, gravure coating, offset printing, flexographic printing, screen printing, and ink-jet printing. The ink composition can also be used as a marking ink. The europium compound represented by the formula (1) in the present invention can be used as an ink composition by using an organic solvent, water or a mixed solvent of water and an organic solvent described later, and can be utilized by being dissolved preferably in water or a mixed solvent of water and an organic solvent. Hereinafter, these compositions are collectively referred to as the aqueous ink composition. The aqueous ink composition of the present invention is used most preferably as ink for ink-jet recording.

Now, the aqueous ink composition of the present invention is described in more detail.

The aqueous ink composition of the present invention is an ink composition comprising a water-soluble europium compound (complex) obtained from a water-soluble phenanthroline derivative, a propanedione derivative and an europium compound, or a europium compound represented by formula (1) , as an essential ingredient and water as a medium (hereinafter, both the water-soluble europium compound (complex) and the europium compound represented by formula (1) are collectively referred to as the europium compound).

The europium compound of the present invention (or a mixture thereof), which is used for preparing the aqueous ink composition of the present invention, is preferably the one with a lower content of inorganic salts. The content, for example in terms of the total content of sodium chloride and sodium sulfate in the compound of the present invention or a mixture thereof, is preferably not higher than 1% by weight, more preferably not higher than 0.5% by weight.

The content of inorganic salts, for example, Cl⁻ and SO₄²⁻ are measured by ion chromatography; heavy metals by an atomic absorption method or ICP (Inductively Coupled Plasma) emission analysis; and Ca²⁺ and Mg²⁺ by ion chromatography, an atomic absorption method or ICP emission analysis.

The europium compound of the present invention can be formed into a coloring matter of a lower content of inorganic salts by stirring a dried product or a wet cake of the europium compound of the present invention, preferably the wet cake, in a solvent (for example a water-containing lower alcohol, preferably a mixed solvent of methanol and water) as necessary, then filtering and drying the compound. Alternatively, the coloring matter with a lower content of inorganic salts can also be obtained by treatment for example with a reverse osmosis membrane.

The europium compound of the present invention can exist in the form of a free acid or a salt thereof. Examples of the usable salt include an alkali metal salt, alkaline earth metal salt, alkyl amine salt, alkanol amine salt or ammonium salt. Preferable examples include an ammonium salt; an alkali metal salt such as sodium salt, potassium salt, lithium salt etc.; and an alkanol amine salt such as monoethanol amine salt, diethanol amine salt, triethanol amine salt, monoisopropanol amine salt, diisopropanol amine salt, triisopropanol amine salt etc.

The aqueous ink composition of the present invention comprises the europium compound of the present invention dissolved in water or a mixed solvent of water and an organic solvent. The pH of the aqueous ink composition is preferably about 6 to 10. This aqueous ink composition can be used as a recording material, and depending on the object, the aqueous ink composition may contain water-soluble high-molecular compounds, ink preparation materials etc. shown later.

As described above, the aqueous ink composition of the present invention is prepared by using water or a mixed solvent of water and an organic solvent as the medium. The amount of the europium compound contained in the ink composition of the present invention, though being varied depending on the field to which it is applied, is usually 0.001 to 10% by weight, preferably 0.01 to 3% by weight, based on the ink composition. The aqueous ink composition of the present invention may contain a water-soluble organic solvent in an amount of not higher than about 90% by weight, preferably not higher than about 70% by weight, more preferably not higher than about 40% by weight, still more preferably not higher than about 30% by weight, and the lower limit may be 0%, but generally the amount of the solvent is not less than about 5% by weight, more preferably not less than about 10% by weight, most preferably 10 to 30% by weight. The aqueous ink composition of the present invention may contain an ink preparation material in an amount of about 0 to 10% by weight, preferably not higher than 5% by weight. The balance other than the components described above is water.

The ink composition of the present invention is prepared by mixing the europium compound of the present invention and, if necessary, the above-described water-soluble organic solvent, ink preparation material etc. with impurity-free water such as distilled water. Alternatively, the europium compound of the present invention may be added to and dissolved in a mixture of water, the water-soluble organic solvent, ink preparation material etc. If necessary, the resulting ink composition may be filtered to remove impurities.

The aqueous ink composition of the present invention contains water. When the ratio of water in ink is increased, conventional ink is made impractical because the solute is precipitated and the emission intensity is deteriorated with time, but the aqueous ink composition of the present invention is excellent in stability even in a mixed solvent containing water in a higher ratio. Accordingly, the aqueous ink composition of the present invention can be ink with smaller environmental problems such as solvent odor and flammability. The drying speed of the ink can be regulated by simultaneously using the water-soluble organic solvent or by changing the amount thereof, wherein the ratio of water in the mixed solvent (water and water-soluble organic solvent) is usually not less than 10% by weight, preferably 20 to 90% by weight, more preferably 30 to 80% by weight.

Now, the aqueous ink composition for ink-jet recording comprising the europium compound of the present invention is described in detail.

The aqueous ink composition for ink-jet recording according to the present invention may contain various water-soluble organic solvents, water-soluble high-molecular compounds, and various ink preparation materials in addition to the europium compound of the present invention and water as the essential ingredients. Further, the aqueous ink composition may contain coloring matters other than the europium compound of the present invention in such a range as not to hinder the object of the present invention.

As described above, the europium compound (which may be a mixture thereof) used in the ink composition for ink-jet recording according to the present invention is preferably the one with a lower content of inorganic salts. The content, for example in terms of the total content of sodium chloride and sodium sulfate in the europium compound of the present invention or a mixture thereof, is preferably not higher than 1% by weight, more preferably not higher than 0.5% by weight.

The water-soluble organic solvents usable in the ink composition for ink-jet recording according to the present invention may be any organic solvents soluble in water, preferably those having a relatively low boiling point.

Examples of the usable water-soluble organic solvents include, for example, C1 to C4 alkanols such as methanol, ethanol, propanol, isopropanol, butanol, isobutanol, secondary butanol, tertiary butanol etc.; lower carboxylic (mono- or di-)lower alkylamides such as N,N-dimethylformamide or N,N-dimethylacetamide; lactams such as ε-caprolactam, N-methylpyrrolidin-2-one etc., preferably 4- to 8-membered lactams; cyclic urea such as 1,3-dimethylimidazolidin-2-one or 1,3-dimethylhexahydropyrimid-2-one, preferably 5- to 6-membered cyclic urea; ketones or ketone alcohols having a linear carbon chain of 4 to 7 carbon atoms such as acetone, methyl ethyl ketone, 2-methyl-2-hydroxypentan-4-one etc.; ethers such as tetrahydrofuran, dioxane etc., preferably 5- to 6-membered cyclic ethers; mono-, oligo- or polyalkylene glycol or thioglycol having a C2 to C6 alkylene unit, such as ethylene glycol, 1,2- or 1,3-propylene glycol, 1,2- or 1,4-butylene glycol, 1,6-hexylene glycol, diethylene glycol, triethylene glycol, dipropylene glycol, thiodiglycol, polyethylene glycol, polypropylene glycol etc.; polyols such as glycerol, hexane-1,2,6-triol etc. (preferably triol having a carbon chain of 3 to 6 carbon atoms); C1 to C4 alkyl ethers of polyvalent alcohols (preferably ethylene glycol or polyethylene glycol), such as ethylene glycol monomethyl ether, ethylene glycol monoethyl ether, diethylene glycol monomethyl ether, diethylene glycol monoethyl ether, triethylene glycol monomethyl ether, triethylene glycol monoethyl ether etc.; and γ-butyrolactone or dimethyl sulfoxide.

Two or more of these water-soluble organic solvents may be simultaneously used. Preferable examples thereof include, for example, N-methylpyrrolidin-2-one, mono-, di- or trialkylene glycol having a C2 to C6 alkylene unit (preferably mono-, di- or triethylene glycol, dipropylene glycol), and particularly N-methylpyrrolidin-2-one, diethylene glycol or dimethyl sulfoxide is preferably used.

The water-soluble high-molecular compounds which can be used in the present invention include, for example, those used conventionally in aqueous ink for jet printing, such as polyethylene glycol, polypropylene glycol, polyvinyl alcohol, anion- or cation-modified polyvinyl alcohol, polyethylene imine, polyvinyl pyrrolidone, a polyvinyl pyrrolidone/vinyl acetate copolymer, a methyl vinyl ether/maleic anhydride copolymer, acryl resin such as base-containing polyacrylate, polymethacrylate, polymethyl methacrylate etc., base-containing polyacrylamide, an acrylamide/base-containing ethyl acrylate copolymer, a styrene/base-containing maleic acid resin, urethane resin etc. Further, modified polyvinyl alcohols such as carboxyl group-modified polyvinyl alcohol, sulfonic acid group-modified polyvinyl alcohol, acetoacetylated polyvinyl alcohol, silica-modified polyvinyl alcohol etc. can also be used.

Particularly preferable examples of water-soluble high-molecular compounds include polymers having at least a cyclic amido group in the structure thereof and carboxyl group- or sulfonic acid group-modified polyvinyl alcohols.

In the present invention, the polymers having at least a cyclic amido group in the structure thereof include, for example, polymers obtained by polymerizing a monomer having a cyclic amido group, such as N-vinyl pyrrolidone, N-vinyl piperidone or N-vinyl caprolactam, or by copolymerizing such a monomer with another monomer. Examples thereof include polyvinyl pyrrolidone, polyvinyl caprolactam, a polyvinyl pyrrolidone/vinyl acetate copolymer, a vinyl pyrrolidone/ vinyl caprolactam copolymer, a vinyl pyrrolidone/vinyl imidazole copolymer, a vinyl pyrrolidone/acrylic acid copolymer, a vinyl pyrrolidone/ methacrylic acid copolymer, a vinyl pyrrolidone/3-methyl-1-vinylimidazolium salt copolymer, and there is no particular limit to the polymers as far as they have a cyclic amido group in the structure thereof. These polymers are preferably those dissolved in water or a mixed solvent of water and a water-soluble organic solvent. The proportion of a monomer having a cyclic amido group in the polymer is preferably 30% or more in terms of molar ratio. In consideration of the stability and viscosity of the aqueous ink, a polymer having a molecular weight of 4000 to 2000000, preferably 5000 to 1000000, more preferably 5000 to 60000, can be used as the polymer having at least a cyclic amido group in the structure. In consideration of the stability and viscosity of the ink during use, the amount of the polymer having a cyclic amido group in the structure is 0.00% to 10%, preferably 0.01% to 3%, based on the aqueous ink composition of the present invention.

As the carboxyl group- or sulfonic acid group-modified polyvinyl alcohol in the present invention, for example the carboxyl group-modified polyvinyl alcohol is obtained through saponification of a vinyl acetate/ethylenically unsaturated dicarboxylic acid and/or unsaturated dicarboxylic acid alkyl ester copolymer by a method described in Japanese Patent Publication No. 48-35779, Japanese Patent Application Laid-Open No. 49-36797 or Japanese Patent Application Laid-Open No. 53-91995. The unsaturated dicarboxylic acid or a derivative thereof is preferably maleic acid, fumaric acid, itaconic acid or an alkyl ester thereof. The sulfonic acid group-modified polyvinyl alcohol is obtained through saponification of e.g. an olefin sulfonic acid (or salt thereof)/vinyl acetate copolymer by a method described in Japanese Patent Application Laid-Open No. 58-179691. In consideration of the stability with time and viscosity of the aqueous ink composition, the usable carboxyl group- or sulfonic acid group-modified polyvinyl alcohol is the one preferably having a degree of polymerization of 150 to 2000, a degree of saponification of 75 to 99 mol-%, and a carboxylic acid group or sulfonic acid group content of 0.01 to 5 mol-% in the copolymer. In consideration of ink stability and viscosity during use, the amount of the carboxyl group- or sulfonic acid group-modified polyvinyl alcohol used is 0.001 to 10% by weight, preferably 0.01 to 3% by weight, based on the aqueous ink composition of the present invention.

When the aqueous ink composition for ink-jet recording according to the present invention is used in prints requiring water resistance, a water-soluble waterproofing agent may be added thereto. Examples of usable waterproofing agents include glyoxal, methylol melamine, water-soluble epoxy compound etc. The water-soluble epoxy compound includes, for example, ethylene polyethylene glycol diglycidyl ether, propylene polypropylene glycol diglycidyl ether, glycerol polyglycidyl ether, lauryl alcohol glycidyl ether etc. The amount of the waterproofing agent is usually 0.5 to 50% by weight, more preferably 1 to 30% by weight based on the amount of the alkali salt in the binder.

As the ink preparation materials, it is possible to use any components usually used as ink preparation materials other than the europium compound of the present invention (coloring matter component), water, the water-soluble organic solvent and the water-soluble high-molecular compound. The ink preparation materials include , for example, an antiseptic and anti-corrosive agent, a pH adjusting agent, a rust preventive, a water-soluble UV absorber, a water-soluble high-molecular compound, a surfactant, a solubilizer, a specific-resistance regulator etc. The antiseptic and anti-corrosive agent includes, for example, sodium dehydroacetate, sodium sorbate, sodium 2-pyridinethiol-1-oxide, sodium benzoate, sodium pentachlorophenol etc. The pH adjusting agent may be any arbitrary substances capable of regulating the pH of the ink in the range of 6 to 10 without adversely affecting the ink prepared. Examples thereof include alkanolamines such as diethanolamine, triethanolamine etc., alkali metal hydroxides such as lithium hydroxide, sodium hydroxide, potassium hydroxide etc., ammonium hydroxide, and alkali metal carbonates such as lithium carbonate, sodium carbonate, potassium carbonate etc. The rust preventive includes, for example, acidic sulfite, sodium thiosulfate, ammonium thioglycolate, diisopropyl ammonium nitrite, pentaerythritol tetranitrate, dicyclohexyl ammonium nitrite etc. The solubilizer includes, for example, urea, ethylene urea, ε-caprolactam etc.

As the specific-resistance regulator, inorganic or organic salts are used.

The aqueous ink composition for ink-jet recording according to the present invention is prepared by using water as the medium. The europium compound (complex) of the present invention is contained in an amount of usually 0.001 to 10% by weight, preferably 0.01 to 3% by weight, in the ink composition. The water-soluble organic solvent may be contained, if desired, in an amount of not higher than about 90% by weight, preferably not higher than 70% by weight, more preferably not higher than 40% by weight, still more preferably not higher than 30% by weight. The lower limit may be 0%, but is generally not lower than 5% by weight, more preferably not lower than 10% by weight, most preferably 10 to 30% by weight. The water-soluble high-molecular compound is contained in an amount of 0.001 to 10% by weight, preferably 0.01 to 3% by weight, in the ink composition. The ink preparation materials may be contained in an amount of about 0 to 10% by weight, preferably not higher than 5% by weight. The balance excluding the above components is water.

The aqueous ink composition for ink-jet recording according to the present invention is prepared by adding the europium compound (complex) of the present invention, the above-described water-soluble organic solvent, the above-described water-soluble high-molecular compound and if necessary the above-described ink preparation materials etc. to impurity-free water such as distilled water. The order of mixing these chemicals is arbitrary. If necessary, the resulting aqueous ink composition for ink-jet recording may be filtered to remove impurities.

The aqueous ink composition of the present invention, including the case where it is used as an aqueous ink composition for ink-jet recording, contains water. When the ratio of water in ink is increased, conventional ink is made impractical because precipitation occurs and the emission intensity is deteriorated with time, but the aqueous ink of the present invention is excellent in stability even in a mixed solvent containing water in a higher ratio. Accordingly, the aqueous ink of the present invention can be ink with smaller environmental problems such as solvent odor and flammability.

Examples of the recording material used in the ink-jet recording method of the present invention include, for example, an information transmission sheet such as paper, film etc.

The information transmission sheet is preferably a surface-treated sheet, specifically a sheet having a base material provided with an ink receiving layer. The ink receiving layer is arranged by impregnating or coating the base material with a cationic polymer or by applying, together with a hydrophilic polymer such as polyvinyl alcohol and polyvinyl pyrrolidone, inorganic fine particles (e.g. porous silica, alumina sol or special ceramics) capable of absorbing the coloring matter in ink, onto the surface of the base material. Further, a fluorescent dye can be applied for improving whiteness. As a matter of course, the ink-jet recording method of the present invention can also be applied to an ordinary paper.

It is known that usually, the information transmission sheet provided with an ink receiving layer can give more vivid images and higher water resistance.

The aqueous ink composition of the present invention is particularly suitable for use in an ink-jet recording system. Examples of the recording material used in the ink-jet recording method include, for example, information transmission sheets such as paper, film etc., and use is made of not only an ordinary paper, synthetic paper and film but also such base materials which are further provided with an ink receiving layer. The ink receiving layer is arranged for example by impregnating or coating the base material with a cationic polymer or by applying, together with a hydrophilic polymer such as polyvinyl alcohol and polyvinyl pyrrolidone, inorganic fine particles (e.g. porous silica, alumina sol or special ceramics) capable of absorbing the coloring matter in ink, onto the surface of the base material. Further, a fluorescent dye can be applied for improving whiteness. The ink receiving layer may also be subjected to color printing. It is known that usually, the information transmission sheet provided with an ink receiving layer can give more vivid images and higher water resistance. The thickness of the dried coating after printing, though being varied depending on a printing system, is preferably about 0.001 to 10 µm (or 0.001 to 10 g/m² in weight), more preferably about 0.005 to 3 µm. Particularly in the case of the ink-jet recording method, about 0.005 to 0.2 µm is preferable.

For recording a recording material by the ink-jet recording method of the present invention, for example a container containing the aqueous ink composition is set in an ink jet printer and used in recording the above-described recording material by a usual method. The ink jet printer includes, for example, printers in a piezo system utilizing mechanical vibration or printers in a bubble jet system utilizing bubbles generated by heating.

The europium compound (complex) of the present invention has high solubility in a water-containing solvent, and can be combined with a water-soluble solvent to prepare an ink with reduced environment problems such as solvent odor and flammability.

The composition comprising the europium compound (complex) of the present invention is substantially invisible in the visible region, and emit red light upon irradiation with UV rays. Printed marks cannot be recognized in usual visible lights, so that they do not deteriorate the outward appearance. Even with the recording material containing a fluorescent dye for improving whiteness, the printed marks can be detected sensitively with a sensor without undergoing the influence of the base material. Accordingly, the europium compound (complex) of the present invention and the ink composition comprising the same can be applied to prints related to hidden letters or security.

The composition comprising the europium compound (complex) of the present invention has high solubility in a water- or water-soluble organic solvent-containing solvent, and is excellent in stability with time in a mixed solvent containing water in a high ratio. Further, the aqueous composition is free of conventional problems such as clogging of nozzles in an ink jet printer due to insufficient stability with time, and sedimentation at the time of coating. Further, prints obtained from the composition comprising the europium compound (complex) of the present invention are excellent in light resistance. In addition, the aqueous ink of the present invention gives a coating excellent in abrasion resistance, water resistance and light resistance.

### Example

Hereinafter, the present invention is described in more detail by reference to the Examples, but the present invention is not limited to the Examples. In the Examples, "parts" refers to parts by weight unless otherwise specified.

### Example 1 (Synthesis of the above compound (4))

50 parts of ethanol, 2.7 parts of 4,4,4-trifluoroalkyl-l-(2-thienyl)-1,3-butane dione, 2.1 parts of sodium 4,7-diphenyl-1,10-phenanthroline disulfonate, and 4.7 parts of 10% caustic soda were mixed at room temperature, and to this solution was dropped an aqueous solution prepared by dissolving 1.4 parts of europium chloride hexahydrate in 30 parts of water, and the mixture was stirred for 2 hours. After the reaction was finished, the solvent was distilled away, and then the resulting white solids were suspended in isopropanol, filtered, washed with water and dried to give 5 parts of the above compound (4).

Absorption spectrum (methanol) absorption maximum wavelength 341 nm

Fluorescence spectrum (methanol) excitation maximum wavelength 341 nm: emission maximum wavelength 616 nm

Decomposition point: about 253°C (measured by TG-DTA, a differential thermal/thermogravimetric measuring device manufactured by Seiko Instruments)

### Example 2 (Synthesis of the above compound (5))

50 parts of ethanol, 3.2 parts of 4,4,4-trifluoroalkyl-1- (2-naphthyl)-1,3-butane dione, 2.1 parts of sodium 4,7-diphenyl-1,10-phenanthroline disulfonate, and 4.7 parts of 10% caustic soda were mixed at room temperature, and to this solution was dropped an aqueous solution prepared by dissolving 1.4 parts of europium chloride hexahydrate dissolved in 30 parts of water, and the mixture was stirred for 2 hours. After the reaction was finished, the solvent was distilled away, and then the resulting white solids were suspended in isopropanol, filtered, washed with water and dried to give 5 parts of the above compound (4).

Absorption spectrum (methanol) absorption maximum wavelength 334 nm

Fluorescence spectrum (methanol) excitation maximum wavelength 336 nm: emission maximum wavelength 616 nm

Decomposition point: about 291°C (measured by the above TG-DTA)

### Example 3

0.3 part of Compound (4), 1 part of ethanol, 93.7 parts of water, and 5 parts of diethylene glycol were mixed and dissolved, and this solution was subjected to microfiltration with a 0.45 µm membrane filter to prepare the aqueous ink composition of the present invention. This ink composition was not separated or precipitated during storage, and even after long-term storage, its physical properties were not changed.

Using an ink jet printer (trade name: PICTY100L, manufactured by NEC), this composition was subjected to ink-jet recording in solid pattern and bar-code pattern on 3 kinds of paper, that is, an ordinary paper (Canon printer paper A4 (TLB5A4S)), special paper A (coated paper for color image jetting STX73A4 (manufactured by Sharp Corporation)), and a kraft paper envelope. Recorded marks could not be recognized in visible rays, but could be confirmed to emit red light well upon irradiation with UV rays. Further, the portion of a bar code printed on special paper A was cut off, then dipped in water for 2 minutes, then air-dried and irradiated with UV rays, to confirm good emission without blurring the bar.

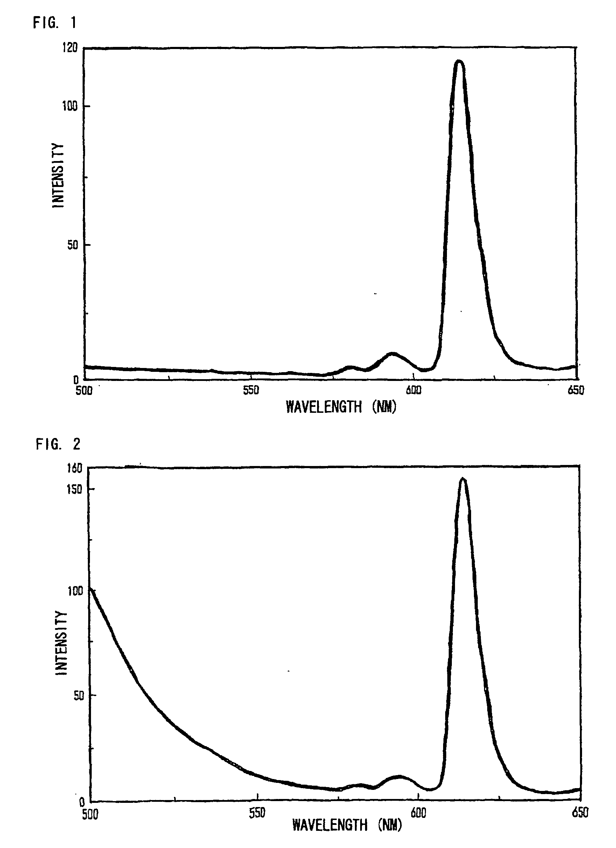

Its fluorescence spectrum was taken by using a fluorospectrophotometer FP-6600 manufactured by JASCO Corporation. The fluorescence spectrum on the ordinary paper is shown in Fig. 1, and the absorption spectrum on the special paper A is shown in Fig. 2.

As can be seen from Fig. 1, a fluorescence peak attributable to Compound (4) appears sharply in the vicinity of 616 nm. As can also be seen from Fig. 2, a fluorescence peak attributable to Compound (4) and a fluorescence peak attributable to a fluorescent dye contained in a coating material in special paper A can be clearly separated and distinguishable from each other.

### Example 4

0.3 part of Compound (4), 10 parts of ethanol, 84.7 parts of water, and 5 parts of diethylene glycol were mixed and dissolved, and this solution was subjected to microfiltration with a 0.45 µm membrane filter to prepare the aqueous ink composition of the present invention. This ink composition was not precipitated or separated during storage, and even after long-term storage, its physical properties were not changed.

Using an ink jet printer (trade name: PICTY100L, manufactured by NEC), this composition was subjected to ink-jet recording in solid pattern and bar-code pattern on 3 kinds of paper, that is, an ordinary paper (Canon printer paper A4 (TLB5A4S)), special paper A (coated paper for color image jetting STX73A4 (manufactured by Sharp Corporation)), and a kraft paper envelope. Recorded marks could not be recognized in visible rays, but could be confirmed to emit red light well upon irradiation with UV rays.

### Example 5

0.3 part of Compound (4), 60 parts of ethanol, 34.7 parts of water, and 5 parts of diethylene glycol were mixed and dissolved, and this solution was subjected to microfiltration with a 0.45 µm membrane filter to prepare the aqueous ink composition of the present invention.

This ink composition was not precipitated or separated during storage, and even after long-term storage, its physical properties were not changed.

Using an ink jet printer (trade name: PICTY100L, manufactured by NEC), this composition was subjected to ink-jet recording in solid pattern and bar-code pattern on 3 kinds of paper, that is, an ordinary paper (Canon printer paper A4 (TLB5A4S)), special paper A (coated paper for color image jetting STX73A4 (manufactured by Sharp Corporation)), and a kraft paper envelope. Recorded marks could not be recognized in visible rays, but could be confirmed to emit red light well upon irradiation with UV rays.

### Example 6

0.3 part of Compound (4), 50 parts of ethanol, 44.7 parts of water, and 5.0 parts of polyvinyl pyrrolidone (molecular weight 38000) (trade name: PVP K-30, manufactured by ISP Japan) were mixed and dissolved, and this solution was subjected to microfiltration with a 0.45 µm membrane filter to prepare the aqueous ink composition of the present invention. This ink composition was not precipitated or separated during storage, and even after long-term storage, its physical properties were not changed.

Using an ink jet printer (trade name: PICTY100L, manufactured by NEC), this composition was subjected to ink-jet recording in solid pattern and bar-code pattern on 3 kinds of paper and film, that is, an ordinary paper (Canon printer paper A4 (TLB5A4S) ) , special paper A (coated paper for color image jetting STX73A4 (manufactured by Sharp Corporation)), and an OHP film.

Recorded marks could not be recognized in visible rays, but could be confirmed to emit red light well upon irradiation with UV rays. Further, the portion of a bar code printed on the OHP film was rubbed with fingers just after printing, but the mark was colored excellently without being rubbed off. Further, the portions of bar codes printed on the ordinary paper, the special paper A and the OHP film were cut off respectively, then dipped in water for 2 minutes, then air-dried and irradiated with UV rays, to confirm good coloration without blurring the bars.

### Example 7

| | |
|---|---|
| Ethanol | 60 parts |
| Water | 40 parts |
| KL-506 (Note 1) | 0.3 part |
| Compound (5) | 0.3 part |
| Note 1: KL-506 (manufactured by Kuraray), carboxyl group-modified PVA (degree of polymerization, 600; degree of saponification, 74 to 80) | |

The above ingredients were mixed and dissolved, and this solution was subjected to microfiltration with a 0.45 µm membrane filter to prepare the aqueous ink composition of the present invention. This ink composition was applied by a bar coater onto white PET Toray Lumilar E22 and dried such that the weight of the resulting coating after drying was about 0.05 g/m² (coating thickness, about 0.05 µm), to give the coating of the ink composition of the present invention.

### Example 8

A coating of the aqueous ink composition of the present invention was prepared in the same manner as in Example 7 except that Compound (4) was used in place of Compound (5) in Example 7.

### Example 9

A coating of the aqueous ink composition of the present invention was prepared in the same manner as in Example 7 except that 0.3 part of KL-506 in Example 7 was changed into 1.2 parts.

### Example 10

A coating of the aqueous ink composition of the present invention was prepared in the same manner as in Example 7 except that Kuraray Poval KL-318 (Note 2) was used in place of KL-506 in Example 7.

Note 2: KL-318 (Kuraray), carboxyl group-modified PVA (degree of polymerization, 1800; degree of saponification, 85 to 90).

### Example 11

A coating of the aqueous ink composition of the present invention was prepared in the same manner as in Example 7 except that Goseran L-3266 (Note 3) was used in place of KL-506 in Example 7.

Note 3: Goseran L-3266 (manufactured by Nippon Gosei), sulfonic acid-modified PVA (degree of polymerization, 300; degree of saponification, about 90).

The ink compositions thus obtained and the coatings of the ink compositions were evaluated, and the results are shown in Table 1. The evaluation criteria used were those described below.

**Table 1**

| Evaluation results | | | | | | |
|---|---|---|---|---|---|---|
| | State of the ink | | Emission intensity | | Abrasion resistance | Water resistance |
| | Just after preparation | With time | Just after preparation | With time | | |
| Example 7 | ○ | ○ | 144 | 139 | ○ | ○ |
| Example 8 | ○ | ○ | 120 | 121 | ○ | ○ |
| Example 9 | ○ | ○ | 180 | 192 | ○ | ○ |
| Example 10 | ○ | ○ | 230 | 215 | ○ | ○ |
| Example 11 | ○ | ○ | 182 | 158 | ○ | ○ |

### Description of each test method

Each test method in Table 1 is as follows.

### (1) State of the ink (just after preparation)

The state of the resulting ink composition was evaluated by observation with the naked eye.
○: Stable in a uniform solution or dispersion state.
Δ: Precipitates were observed, but the ink was excellent in re-dispersibility and made uniform by stirring.
×: Precipitated and separated.

### (2) State of the ink (with time)

The resulting ink composition was stored at 40°C for 2 weeks, and the state of the ink was observed with the naked eye, to evaluate stability.
○: Stable in a uniform solution or dispersion state.
Δ: Precipitates were observed, but the ink was excellent in re-dispersibility and made uniform by stirring.
×: Precipitated and separated.

### (3) Emission intensity (just after preparation)

The resulting coating of the ink composition was measured for emission intensity. This measurement was conducted using a spectrofluorophotometer FP-6600 (manufactured by JASCO Corporation). For this measurement, the coating was irradiated with excitation light at 320 nm and measured for emission intensity at 615 nm.

### (4) Emission intensity (with time)

The resulting ink composition was stored at 40°C for 2 weeks, and the emission intensity of the coating was measured in the same manner as in (3).

### (5) Abrasion resistance

The resulting coating of the ink composition was rubbed once (both ways) with fingers and then irradiated with UV rays to confirm emission.
○: Good red coloration
×: No coloration

### (6) Water resistance

The resulting coating of the ink composition on each base material was dipped in tap water for 1 minute, then dried and irradiated with UV rays to confirm emission.
○: Good red coloration
×: No coloration or weak coloration

As is evident from Table 1, the aqueous ink of the present invention was excellent in stability without a reduction in emission intensity, and the coating thereof was excellent in abrasion resistance and water resistance.

### Example 12

| | |
|---|---|
| Ethanol | 60 parts |
| Water | 40 parts |
| K-43 (Note 4) | 0.3 part |
| Compound (5) | 0.3 part |
| Note 4: K-43 (manufactured by BASF), polyvinyl Caprolactam | |

The above ingredients were mixed and dissolved, and this solution was subjected to microfiltration with a 0.45 µm membrane filter to prepare the aqueous ink composition of the present invention. This ink composition was applied by a bar coater onto white PET Toray Lumilar E22 and dried such that the weight of the resulting coating after drying was about 0.05 g/m² (coating thickness, about 0.05 µm), to give the coating of the aqueous ink composition of the present invention.

### Example 13

A coating of the aqueous ink composition of the present invention was prepared in the same manner as in Example 12 except that Compound (4) was used in place of Compound (5) in Example 12.

### Example 14

A coating of the aqueous ink composition of the present invention was prepared in the same manner as in Example 12 except that 0.3 part of K-43 in Example 12 was changed into 1.2 parts.

### Example 15

A coating of the aqueous ink composition of the present invention was prepared in the same manner as in Example 12 except that VA-64 (Note 5) was used in place of K-43 in Example 12.

Note 5: VA-64 (manufactured by BASF), a vinyl pyrrolidone/vinyl acetate copolymer

### Example 16

A coating of the aqueous ink composition of the present invention was prepared in the same manner as in Example 12 except that VPC55K65 (Note 6) was used in place of K-43 in Example 12.

Note 6: VPC55K65 (manufactured by BASF), a vinyl pyrrolidone/vinyl caprolactam copolymer

### Example 17

A coating of the aqueous ink composition of the present invention was prepared in the same manner as in Example 12 except that VPI55K72 (Note 7) was used in place of K-43 in Example 12.

Note 7: VPI55K72 (manufactured by BASF), a vinyl pyrrolidone/vinyl imidazole copolymer

Each aqueous ink composition thus obtained and the coating of the ink composition were evaluated, and the results are shown in Table 2. The test methods are identical with those described above.

**Table 2**

| Evaluation results | | | | | | |
|---|---|---|---|---|---|---|
| | State of the ink | | Emission intensity | | Abrasion resistance | Water resistance |
| | Just after preparation | With time | Just after preparation | With time | | |
| Example 12 | ○ | ○ | 237 | 210 | ○ | ○ |
| Example 13 | ○ | ○ | 164 | 152 | ○ | ○ |
| Example 14 | ○ | ○ | 205 | 214 | ○ | ○ |
| Example 15 | ○ | ○ | 148 | 154 | ○ | ○ |
| Example 16 | ○ | ○ | 162 | 158 | ○ | ○ |
| Example 17 | ○ | ○ | 179 | 146 | ○ | ○ |

As is evident from Table 2, the aqueous ink of the present invention was excellent in stability without a reduction in emission intensity, and the coating thereof was excellent in abrasion resistance and water resistance.

### Industrial Applicability

The europium compound (complex) of the present invention and the aqueous ink composition comprising the same are invisible to the naked eye in the visible range, but emit red light upon irradiation with UV lights. Printed marks cannot be recognized in usual visible lights and do thus not deteriorate the outward appearance, and a recording medium even containing a fluorescent dye for improving whiteness can be detected sensitively with a sensor without undergoing the influence of a substrate (fluorescent dye). Accordingly, the aqueous ink composition of the present invention can be applied to prints requiring hidden letters and security in a system having a sensor reading function.

The europium compound (complex) of the present invention and the ink composition comprising the same are excellent in stability and superior in solubility in a solvent, particularly in a water-containing solvent. Recording with the ink composition of the present invention is excellent in light resistance.

## Claims

1. A europium compound represented by formula (1): 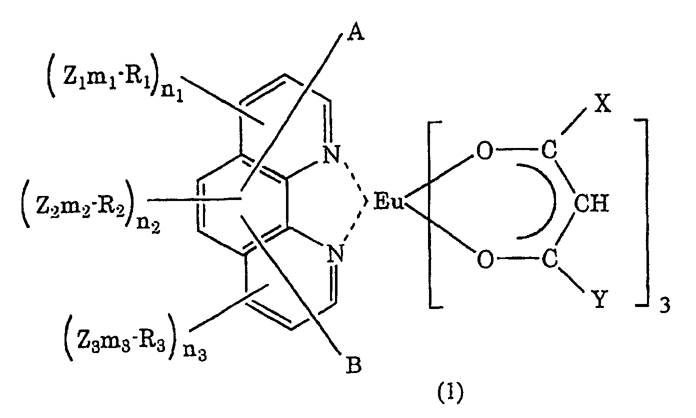 wherein X represents an aromatic or heterocyclic group which may have a substituent group, Y represents a fluorohydrocarbon group having 1 to 10 carbon atoms, A and B independently represent a substituent group, Z₁ to Z₃ independently represent a hydrophilicity-imparting group, R₁ to R₃ are each independently a direct bond or an optionally substituted divalent hydrocarbon group or heterocyclic group, n₁ to n₃ are each independently an integer of 0 to 2, and m₁ to m₃ are each independently an integer of 1 to 3, with the proviso that all of n₁ to n₃ are not simultaneously 0.

2. The europium compound according to claim 1, wherein each of Z₁ to Z₃ is a group selected from the group consisting of a sulfonic acid group, a carboxyl group, a hydroxyl group and a phosphonic acid group.

3. The europium compound according to claim 1 or 2, wherein each of R₁ to R₃ is a phenyl group.

4. The europium compound according to any one of claims 1 to 3, wherein each of Z₁ to Z₃ is a sulfonic acid group, and each of R₁ to R₃ is a phenyl group.

5. A europium compound represented by formula (2): 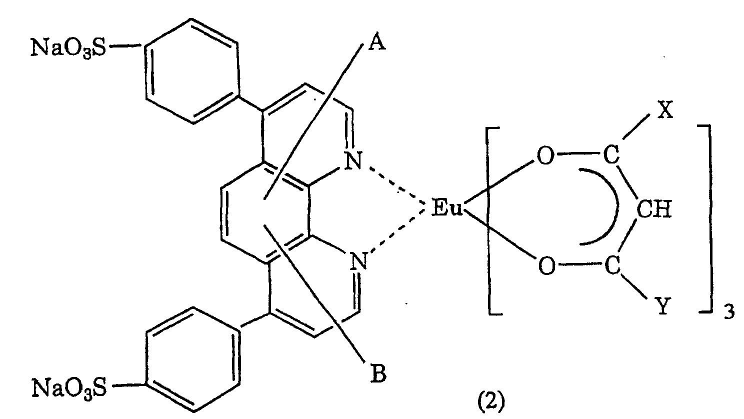 wherein X represents an aromatic or heterocyclic group which may have a substituent group, Y represents a fluorohydrocarbon group having 1 to 10 carbon atoms, and A and B independently represent a substituent group.

6. The europium compound according to claim 5, wherein in the formula (2), X is a group selected from the group consisting of a benzene ring group, a naphthalene ring group, a pyridine ring group, a thiophene ring group and a furan ring group, each of which may have a substituent group.

7. The europium compound according to claim 5 or 6, wherein in the formula (2), Y is a trifluoromethyl group.

8. The europium compound according to claim 5, wherein in the formula (2), the substituent groups A and B are each independently a hydrogen atom or a hydrocarbon group having 1 to 8 carbon atoms which may have a substituent group.

9. A europium compound represented by formula (3): 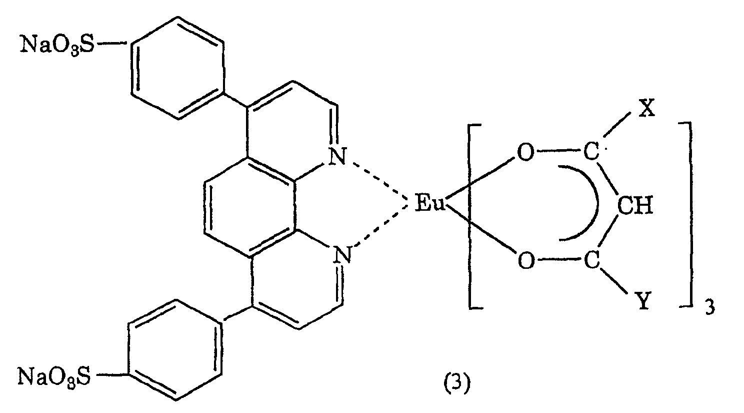 wherein X represents an aromatic or heterocyclic group which may have a substituent group, and Y represents a fluorohydrocarbon group having 1 to 10 carbon atoms.

10. The europium compound according to claim 9, wherein in the formula (3), X is a group selected from the group consisting of a benzene ring group, a naphthalene ring group, a pyridine ring group, a thiophene ring group and a furan ring group, each of which may have a substituent group.

11. An aqueous ink composition comprising a water-soluble europium compound (complex) obtained from a water-soluble phenanthroline derivative, a propanedione derivative and a europium compound.

12. An aqueous ink composition, which comprises the europium compound according to any one of claims 1 to 10.

13. The aqueous ink composition according to claim 11 or 12, which comprises an organic solvent.

14. The aqueous ink composition according to any one of claims 11 to 13, which comprises a polymer having a cyclic amido group.

15. The aqueous ink composition according to claim 14, wherein the polymer having a cyclic amido group is at least one member selected from the group consisting of polyvinyl caprolactam, a vinyl pyrrolidone/vinyl caprolactam copolymer, a vinyl pyrrolidone/vinyl imidazole copolymer, polyvinyl pyrrolidone, and a vinyl pyrrolidone/vinyl acetate copolymer.

16. The aqueous ink composition according to claim 11 or 13, which comprises a carboxyl group- or sulfonic acid group-modified polyvinyl alcohol.

17. The aqueous ink composition according to any one of claims 11 to 16, which is used in ink-jet recording.

18. An ink-jet recording method for recording a recording material by discharging ink droplets depending on record signal, **characterized by** using the aqueous ink composition according to any one of claims 11 to 17.

19. The ink-jet recording method according to claim 18, wherein the recording material is an information transmission sheet.

20. The ink-jet recording method according to claim 19, wherein the information transmission sheet is a surface-treated sheet.
